# EUROPEAN PATENT APPLICATION

(11) **EP 1 739 586 A2**
(43) Date of publication of application: **03.01.2007**
(21) Application number: 06253373.2
(22) Date of filing: 28.06.2006
(51) Int. Cl.: G06F 19/00

(54) **Apparatus & method for donducting large scale clinical trials**

(30) Priority: 29.06.2005 US 170297
(71) Applicant: JOHNSON & JOHNSON CONSUMER COMPANIES, INC., Skillman, NJ 08558 (US)
(72) Inventor: Cole, Curtis A., Ringoes, NJ 08551 (US); Kollias, Nikiforos, Skillman, NJ 08558 (US); Payonk, Gregory, Flanders, NJ 07836 (US)
(74) Representative: Tunstall, Christopher Stephen

(57) **Abstract**

A system for conducting large scale clinical trials has a plurality of data collection nodes for collecting data from subjects participating in the clinical trial. A data analysis facility is located remotely from the data collection nodes and the data is communicated from the data collection nodes to the data analysis facility, where it is stored and analyzed. Each data collection node may include testing and data entry facilities, e.g., in the form of photographic equipment or various diagnostic and measuring probes, such as those used to measure skin color, elasticity and moisture content. The data nodes may be operated by the participant.

## Description

### Field of the Invention

The present invention relates to apparatus and methods for conducting clinical trials, and more particularly, for conducting such trials on a large scale and in an economical manner.

### Background of the Invention

Clinical trials for testing the efficacy of medicaments, therapeutic compounds and treatments are typically conducted in controlled clinical settings. This approach is clearly required for the testing of any agent or course of treatment that has any potential for negative side effects or is unknown as to its effects. Due to the rigorous procedures, complex equipment and highly trained personnel employed to conduct traditional clinical trials, they are expensive and complex. The number of subjects tested directly multiplies costs. In order to avoid excessive costs, traditional clinical trials usually only test a relatively small sample population, thus limiting the amount of data that is gathered and the predictive value of the testing. There are products, therapeutic agents and treatments, however, that are known to be benign. For example, in the area of skin treatments, many lotions, washes and creams are essentially harmless and therefore do not really require the rigorous procedures utilized in typical clinical trials to protect the test population. Besides limiting the size of the test population, laboratory based clinical trials inadvertently eliminate an important factor which impacts the efficacy of therapeutic agents, viz., by closely controlling the behavior of the test population relative to the use of the agent, the trial masks the actual efficacy of the agent as it will be used in the real world, i.e., by the user population when they are not subject to the rules and disciplines of the clinical setting. Accordingly, it would be beneficial to have a clinical testing apparatus and methods which overcome the foregoing limitations.

### Summary of the Invention

The problems and disadvantages associated with conventional apparatus and techniques utilized to conduct clinical trials are overcome by the present invention, which includes a system for conducting large scale clinical trials including a plurality of data collection nodes for collecting data from subjects participating in a clinical trial. A data analysis facility is located remotely from the data collection nodes and the data is communicated from the data collection nodes to the data analysis facility, where it is stored and analyzed.

### Brief Description of the Drawings

FIG. 1 is a schematic diagram of a system for conducting clinical trials in accordance with an embodiment of the present invention; and
FIG. 2 is a flowchart of exemplary procedures conducted in accordance with an embodiment of the present invention;

### Detailed Description of the Invention

The present invention may be utilized for testing various products and/or treatments. For the purposes of describing the present invention, the example of testing skin products and treatments is employed herein. This is just one of many types of products and treatments which could be tested. The monitoring and maintenance of healthy skin is an important concern for most people. Typically people examine their skin using a mirror in a setting with natural, incandescent and/or fluorescent lighting. This self examination process is used by a person to ascertain the condition of their skin and potentially to treat the skin with various therapies and preparations in order to improve the condition of the skin. For example, upon viewing the skin in the mirror and ascertaining that the skin looks oily, the selection and use of a washing and/or drying agent may be employed. The presence of wrinkled skin may indicate that a moisturizer or other wrinkle treatment would be advisable. People with acne frequently check their skin in the mirror to monitor and treat the acne condition. In addition to the skin conditions that are readily visible in normal lighting environments, there are also conditions that are invisible to inspection using a mirror in typical lighting. For example, subsurface conditions of the skin, such as the dilation of blood vessels below the surface, and UV photo damage to subsurface layers (mainly due to exposure to the sun), etc., will not necessarily be apparent by simply viewing the surface of the skin in a mirror. It is now known that inspection of the skin utilizing various wavelengths of light and/or polarized light can illuminate and reveal skin conditions which would otherwise be imperceptible. In addition, these alternative illuminating techniques can highlight and emphasize visible conditions, such as wrinkles or acne. Known techniques for sub-surface or enhanced surface viewing typically involve photography, wherein a flash unit which is capable of producing light of a particular wavelength is activated and an image captured with a camera. Various filters may also be employed in this process. For example, polarized photography has been utilized to enhance the surface or subsurface features of the skin by placing the polarizer in front of a flash unit and in front of a camera, and a photograph of the skin taken under these conditions. When the pictures obtained are examined, surface features of the skin, such as scales, wrinkles, fine lines, pores, and hairs are visually enhanced. When the polarizers are arranged perpendicular to each other, subsurface features of the skin such as erythema pigmentation and blood vessels are visually enhanced. When the polarizers are in the same orientation, surface features of the skin such as scales, wrinkles, fine lines, pores and hairs are visually enhanced. Ultraviolet (UV) photography utilizing a flash unit filtered to produce ultraviolet A light and a camera is filtered so that only visible light enters the lens produces images that are visually enhanced with regard to pigmentation, the presence of the bacteria p. acnes and horn. A variation of ultraviolet photography has been termed the "sun camera" where ultraviolet A light is used to illuminate the skin and an ultraviolet A sensitive digital camera is used to record the ultraviolet light reflected from the skin. In this arrangement, both pigment distribution and the surface features of the skin are visually enhanced.

The following United States Patent Applications, all of which are owned by and assigned to the assignee of the present application, disclose apparatus and methods for examining the condition of the skin and for recording data representing the condition of the skin: U.S. Application No. (to be assigned), entitled Skin Imaging System with Probe, filed concurrently herewith; U.S. Application No. (to be assigned), entitled Handheld Device for Determining Skin Age, Proliferation Status and Photodamage Level, filed concurrently herewith; U.S. Application No. (to be assigned), entitled Apparatus and Method for Viewing the Skin, filed concurrently herewith; and U.S. Application No. 10/978,284, entitled Apparatus and Method of Taking and Viewing Images of the Skin, filed October 29, 2004. Each of the foregoing applications are incorporated in their entirety herein. The apparatus and methods of the applications incorporated by reference facilitate a consumer to examine/test their own skin and capture data indicative of a variety of skin conditions/qualities, e.g., in the form of digitally stored images, spectrometer and conductance readings and subjective grading based upon observation (e.g., subject self rates skin dryness on a scale of one to ten). This digital information is readily communicable, e.g., over a network, and therefore may be utilized in accordance with the present invention to conduct a clinical trial, as shall be described below.

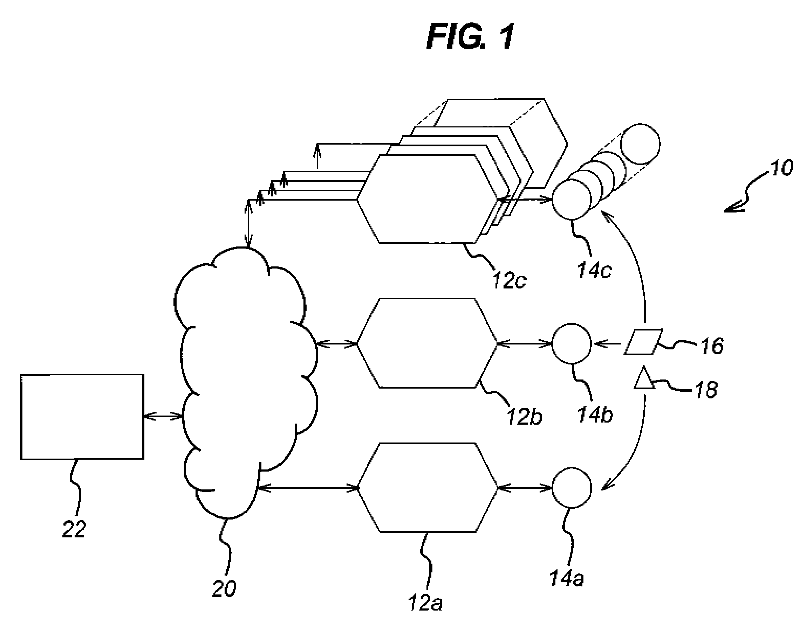

FIG. 1 shows a diagram of the system 10 for conducting clinical trials, which includes a plurality of data collection nodes 12a, b, c where the testing and data collection pertaining to subjects 14a, b, and c occur. As shown in FIG. 1, the system 10 is designed to accommodate any number of data collection nodes 12 to service any number of users 14. The data collection nodes 12 may have various capabilities in terms of testing and data collection. For example, an imaging/testing station, as is disclosed in an application incorporated herein by reference entitled Skin Imaging System with Probe discloses an apparatus capable of visible, polarized and fluorescent photography, as well as touch probes for collecting data on skin color, fluorescence, reflectance, moisturization, firmness, pH, density and elasticity. This data may be interpreted to measure skin proliferation status, physiological age, and the extent of phtotodamage. (It may also be desirable to include 3D imaging for evaluating 3D contours of the subject.) Another example of a data collection node would be the publicly known Neutrogena kiosks that are in use in various shopping malls in the United States. These kiosks have photographic capabilities using various wavelengths of light as well as polarized and fluorescent photography. While public, multi-user data collection nodes 12 have been described, private data collection nodes 12 would also be useful for the present invention. More particularly, a subject could measure skin parameters using a hand-held testing device (e.g., as disclosed in the application entitled Handheld Device for Determining Skin Age, Proliferation Status and Photodamage Level which is incorporated herein by reference and is being filed concurrently herewith) and then enter that data into a home computer. Even more simply, a subject participating in a clinical study in accordance with the present invention may use subjective, visual or tactile self examination and grading, for example on a scale of one to ten, on a computer input screen which solicits data pertaining to the clinical study. Apparatus, such as disclosed in the application entitled Apparatus and Method for Viewing the Skin, which is incorporated herein by reference, would be suitable to enhance the subject's ability to observe characteristics of their skin, and then record those characteristics, e.g., on a scale of one to ten for entry into a computer. In each instance, the data collection nodes 12 are linked to a network 20 or a means of communication which is then accessible to a data analysis facility 22. The data analysis facility 22 may be a computer programmed to receive data in a specific format, which data is then stored, e.g., in tables of a database. The data may be analyzed by queries to the database, numerical/mathematical/graphical analysis or merely printed or displayed for manual analysis. The network 20 can be in the form of the Internet, which is commonly used to receive data inputs from multiple computers in diverse locations. As yet a further alternative, a clinical study could be conducted using the public telephone network wherein subjects conduct examination/testing of their skin and then enter the resultant information into the system via the public telephone network, e.g., by calling a toll free number and keying in the information in response to verbal cues. The data analysis facility 22 can download the data received from the data collection nodes 12 and analyze it according to clinical protocols. For example, the analysis of photographic records may include evaluation of wrinkles, wrinkle length, extent and size of pigmentation, surface texture, redness/irritation and skin laxity of the subjects photographed. Analysis from instrumental probes would include skin color, pigmentation, collagen damage, skin physiological age, elasticity, moisturization, and firmness. Collated data from the same time points may be summarized against base line evaluations to track and determine treatment progress and significance. Many of the parameters potentially measured, for example using touch probes to determine moisture content or skin elasticity and/or spectrometer readings, are all numerically quantifiable and therefore subject to programmatic numerical, graphical and statistical analysis by a computer. As shown in FIG. 1, the process of conducting the clinical trial may be initiated by the dissemination of an advertisement 16, e.g., on the television, radio or in targeted written ads to consumers and potential subjects for the clinical study. This promotional activity may also include the distribution of sample product 18, either in conjunction with the advertisement 16 or upon the consumer enrolling to participate in the clinical study. The data collection nodes 12 may be located in shopping malls, pharmacies, drug stores, medical office centers and are otherwise available to the public. Alternatively, as described above, the consumer's home computer or phone and associated instrumentation in the form of probes or other instrumentalities for testing the skin can be utilized as the data collection node 12. Given that there are numerous data collection nodes 12, potentially distributable and/or already in the field, a mass clinical study can be conducted with low cost. Furthermore, the study conducted will utilize data collected as a consequence of typical behavior patterns of the consumer in their day-today living, rather than under the constraints and unusual disciplines imposed by a lab-based clinical study.

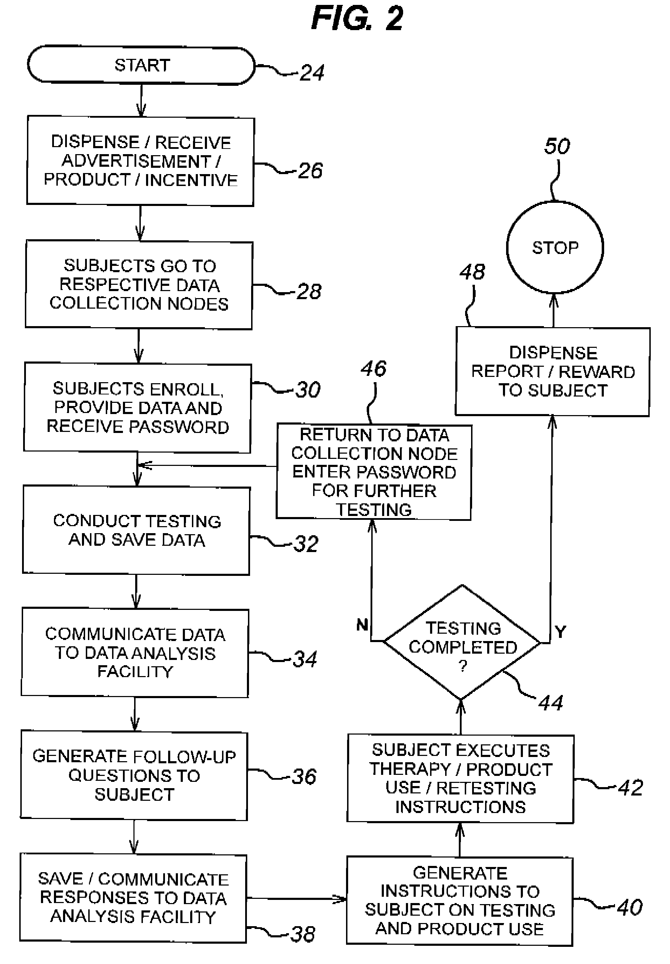

FIG. 2 shows a flow diagram illustrating steps in a method in accordance with the present invention, for conducting a clinical trial. At the start 24, advertisements and/or product samples are dispensed to and received by 26 consumers in an attempt to solicit their participation in the clinical trial. Part of the solicitation may be an instruction for those who are interested to contact the product test host to obtain information regarding the clinical trial, and identifying the location of data collection nodes 12 for capturing the initial base line observations and/or subsequent study data. Through some means, a subset of the consumers solicited will accept the invitation to participate in the clinical trial and the subjects then go 28 to the respective data collection nodes 12. At the data collection nodes 12, the subjects enroll 30 in the study by providing necessary data such as their age, race, occupation, and other information that would be pertinent to ascertaining the characteristics of their skin. The test subjects may be requested to answer a survey or questionnaire concerning their experiences, such as number of hours of exposure to sunlight over the course of their life, their particular observations as to the condition of their skin as to oiliness, wrinkles, dryness, scaliness, etc. After having provided 30 the necessary data, the subjects receive a password that can be used to access the data collection node 12 in the future and to allow the system 10 to properly group the data collected with the specific individual subject. The testing is then conducted, 32, e.g., by having the subject undergo a photographic session, by exposing their skin to various testing probes, and or by viewing their skin in various lights and entering their observations concerning the status of various parameters, e.g., on a scale of one to ten. This testing data is saved and then communicated 34 to the data analysis facility 22, e.g., by way of the network 20. The data analysis facility 22 may have a continuously running program (website) for receiving the communications from the data collection nodes 12, and in response to the receipt of certain data, may pose 36 certain follow-up questions to the subject. The answers to the follow up questions are saved 38 and communicated to the data analysis facility 22. The user is then instructed 40 on further testing and product use. If the data collection node 12 is located in a public facility, it may be staffed by technicians and or other personnel who can assist the subjects to undergo testing and answer the various questions that are posed by the system 10. Further, the staff who may be present at the data collection nodes 12 can provide their own data input for verification of the subjects' data. This verification data can be used to assess how accurate subjects are at evaluating their conditions and in complying with the rules and regimens of the clinical trial. Having conducted the testing and received the various instructions at the data collection node 12, the subject then returns to their normal routine and executes the therapy 42 and/or uses the product that is the subject of the clinical trial in accordance with the retesting instructions received. If the testing is not completed at this cycle 44 in the clinical trial, the subject returns 46 to the data collection node 12 and reenters the password for further testing iterations. When the subject completes the required number of testing interactions, a report may be dispensed 48 to the subject concerning the results obtained as revealed by their testing and/or the subject may be provided with a reward for having participated in the clinical study. The process is then completed 50.

It is understood that while the invention has been described in conjunction with the detailed description thereof, that the foregoing description is intended to illustrate and not limit the scope of the invention, which is defined by the scope of the appended claims. Other aspects, advantages, and modifications are within the scope of the claims.

## Claims

1. A system for conducting large scale clinical trials, comprising:
a plurality of data collection nodes for collecting data from subjects participating in a clinical trial;
a data analysis facility located remotely from said data collection nodes;
means for communicating data between said data collection nodes and said data analysis facility.

2. The system of Claim 1, wherein a plurality of said data collection nodes include means for testing for determining said data.

3. The system of Claim 2, wherein said plurality of said data collection nodes permit the participants in the clinical trial to self-test using said means for testing.

4. The system of Claim 3, wherein said data relates to the skin of the participants in the clinical trial.

5. The system of Claim 4, wherein said data is in the form of photographs.

6. The system of Claim 5, wherein said photographs are taken using light of at least one of white, polarized, blue and UV.

7. The system of Claim 4, wherein said data is in the form of skin measurements of at least one of color, fluorescence, reflectance, moisture content, firmness and elasticity.

8. The system of Claim 1, wherein said means for communication is a network.

9. The system of Claim 8, wherein said network is the Internet.

10. The system of Claim 8, wherein said network is the public telephone system.

11. The system of Claim 1, wherein said data analysis facility includes a computer with database processing capability.

12. The system of Claim 1, wherein said data collection node permits the entry of data by a participant in the clinical study.

13. A method for conducting large scale clinical trials, comprising:
(A) collecting data from subjects participating in a clinical trial at a plurality of data collection nodes;
(B) communicating the data from the data collection nodes to a data analysis facility located remotely from said data collection nodes; and
(C) analyzing the data.

14. The method of Claim 13, wherein said data collection nodes includes means for testing the participants and further including the step of testing the participants to obtain at least a portion of the data prior to said step of communicating.

15. The method of Claim 14, wherein said step of testing is conducted by the participants on themselves.

16. The method of Claim 15, wherein said step of testing includes photographing the participant who conducts the testing.

17. The method of Claim 16, wherein said step of photographing includes taking photographs in light of at least one of white, polarized, blue and UV.

18. The method of Claim 15, wherein said step of testing includes testing the skin of the participant for color, fluorescence, reflectance, moisture content, firmness and elasticity.

19. The method of Claim 15, wherein said step of testing includes testing the skin of the participant with touch probes to measure at least one of skin proliferation status, skin physiological age, extent of photodamage, skin pH and skin density.

20. The method of Claim 13, wherein said step of analyzing includes the step of data extraction and mathematical analysis.

21. The method of Claim 13, wherein said step of analysis includes the step of graphical and statistical analysis to determine treatment performance.
